# EUROPÄISCHE PATENTSCHRIFT

(11) **EP 1 609 800 B1**
(45) Veröffentlichungstag und Bekanntmachung des Hinweises auf die Patenterteilung: **14.11.2007**
(21) Anmeldenummer: 04405395.7
(22) Anmeldetag: 25.06.2004
(51) Int. Cl.: C07K 1/34

(54) **Verfahren zur Extraktion von biologischem Material**
Process for extraction of biological material
Procédé d'extraction d'une matière biologique

(43) Veröffentlichungstag der Anmeldung: 28.12.2005
(73) Patentinhaber: KÜHNI AG, CH-4123 Allschwil (CH)
(72) Erfinder: Riedl, Wolfgang, 79576 Weil am Rhein (DE); Bühlmann, Ulrich, 4105 Biel-Benken (CH); de Bruyn Ouboter, Dirk, 4107 Ettingen (CH)
(74) Vertreter: Roshardt, Werner Alfred

(56) Entgegenhaltungen:
- EP-A- 0 246 065
- WO-A-00/68260
- SIVARS U & TJERNELD F: "Mechanisms of phase behaviour and protein partitioning in detergent/polymer aqueous two-phase systems for purification of integral membrane proteins" BBA - GENERAL SUBJECTS, ELSEVIER SCIENCE PUBLISHERS, NL, Bd. 1474, Nr. 2, 6. April 2000 (2000-04-06), Seiten 133-146, XP004276549 ISSN: 0304-4165
- GROSSMANN C ET AL: "PARITIONING OF LOW MOLECULAR COMBINATION PEPTIDES IN AQUEOUS TWO-PHASE SYSTEMS OF POLY(ETHYLENE GLYCOL) AND DEXTRAN IN THE PRESENCE OF SMALL AMOUNTS OF K2HPO4/KH2PO4 BUFFER AT 293 K: EXPERIMENTAL RESULTS AND PREDICTIONS" BIOTECHNOLOGY AND BIOENGINEERING, INTERSCIENCE PUBLISHERS, LONDON, GB, Bd. 60, Nr. 6, Dezember 1998 (1998-12), Seiten 699-711, XP002930073 ISSN: 0006-3592

## Beschreibung

### Technisches Gebiet

Die Erfindung betrifft ein Verfahren zur Extraktion von Proteinen oder Peptiden, aus einer ersten wässrigen Lösung in eine zweite wässrige Lösung, wobei zwischen den wässrigen Lösungen eine poröse Membran angeordnet ist. Die Erfindung betrifft weiter die Verwendung eines biokompatiblen Stoffes in einem Verfahren zur Extraktion von Proteinen oder Peptiden.

### Stand der Technik

Die Isolation und Feinreinigung von biologischem Material, wie z.B. von Proteinen, Peptiden oder anderen chemisch, biologisch, bio- oder gentechnisch hergestellten Substanzen, die gegenüber Pflanzen und/oder Lebewesen eine biologische Wechselwirkung ("biologische Aktivität") aufweisen, erfordert in der Regel mehrstufige Trennverfahren und verursacht erhebliche Kosten.

Da solche Produkte oft nur in wässrigen Lösungen innerhalb enger Grenzen von Temperatur, pH-Wert und Ionenstärke stabil sind, bietet die in der Biotechnologie gut erforschte Methode der Extraktion mit wässrigen Zwei-Phasen-Systemen ("aqueous two-phase systems", ATPS) häufig ein geeignetes Aufarbeitungsverfahren. Informationen dazu finden sich beispielsweise in C. Grossmann, Untersuchungen zur Verteilung von Aminosäuren und Peptiden auf wässrige Zwei-Phasen-Systeme, Dissertations Druck Darmstadt, Kaiserslautern, 2002 oder J. Brenneisen, Zur Verteilung von Proteinen auf wässrige Zwei-Phasen-Systeme, Der Andere Verlag, Osnabrück, 2001.

Verfahren zur Extraktion von Membranproteinen und von hydrophilen Proteinen (wie BSA oder Lysozym) in wässrigen Zwei-Phasen-Systemen sind im Artikel "Mechanisms of phase behaviour and protein partitioning in detergent/polymer aqueous two-phase systems for purification of integral membrane proteins" (Biochimica et Biophysica Acta 1474, 2000) von U. Sivars und F. Tjerneld beschrieben. Die Systeme bestehen aus Detergens/Polymer/Wasser. Im Rahmen der beschriebenen Versuche wurden zunächst die Zwei-Phasen-Systeme in Reagenzgläsern vorbereitet, dann das zu extrahierende Protein zugefügt, worauf die Systeme eine gewisse Zeit einer vorgegebenen Temperatur ausgesetzt wurden; nach einem erneuten Mischen erfolgte eine Zentrifugation und eine Aufteilung in die obere, Detergens-reiche Phase und die untere, Polymer-reiche Phase. Besonders für Membranproteine ergibt sich eine deutliche Anreicherung in der oberen, Detergens-reichen Phase, während sich die hydrophilen Proteine tendenziell in der unteren, Polymer-reichen Phase sammeln.

Die Herstellung von ATPS erfolgt durch das Mischen wässriger Lösungen zweier wasserlöslicher, aber nicht miteinander mischbarer Polymere. Nach dem Mischen bilden sich zwei Phasen aus. Das bisher wohl am besten untersuchte ATPS ist Polyethylenglykol (PEG) /Dextran. Beide Polymere sind unendlich mit Wasser mischbar. Werden sie allerdings zusammen gegeben, erfolgt ab einer gewissen Polymerkonzentration eine Phasentrennung, wobei sich eine PEG-reiche und eine Dextran-reiche Phase bilden. Dasselbe Phänomen kann auch mit anderen Stoffpaarungen, wie z.B. PEG / Phosphatpuffer erreicht werden.

Der Vorteil der ATPS liegt in den wasserähnlichen Bedingungen der beiden Phasen. So bestehen diese oftmals aus mehr als 80% Wasser. Die biologisch aktiven Komponenten können so schonend extrahiert werden, ohne dabei ihre Aktivität (z.B. durch Denaturierung in Folge von sog. Entfaltung) zu verlieren. Mit geeigneten Puffersystemen kann sogar der pH-Wert der Phasen beliebig eingestellt werden, wodurch wiederum eine Denaturierung (z.B. im stark sauren Milieu) wirksam verhindert werden kann. Nebst dem pH-Wert stehen weitere Faktoren wie Konzentration der phasenbildenden Komponenten, Temperatur, Poly mermolekulargewicht, Salze etc. zur Verfügung, um diese multivariablen Systeme optimal auf die Trennaufgabe abzustimmen. Ausserdem können mit derartigen ATPS die gerade bei Fermentationsprozessen oft störenden Zellreste separat abgetrennt werden, weil sie sich bevorzugt in einer der beiden Phasen anreichern.

Der Nachteil wässriger Zwei-Phasen-Systeme liegt aber darin, dass kaum Unterschiede in den physikalischen Eigenschaften der beiden Phasen feststellbar sind. Insbesondere der Dichteunterschied der beiden wässrigen Phasen ist aufgrund des hohen Wasseranteils (überwiegend mehr als 80 Gew.%) sehr gering (weniger als 60 kg/m³). Ein hinreichend grosser Dichteunterschied (typisch mehr als 80 kg/m³) ist jedoch erforderlich, um die beiden Phasen im Schwerkraftfeld vollständig trennen zu können. Bei einer geringeren Dichtedifferenz, wie sie bei der Extraktion von Wertkomponenten unter Verwendung wässriger Zwei-Phasen-Systeme auftritt, muss deshalb in der Regel eine Zentrifugation erfolgen, siehe beispielsweise K. Sattler, Thermische Trennverfahren, Verlag VCH, Weinheim, 1995.

Die Zentrifugentechnik ist jedoch aufgrund der für die Erzeugung von hohen Zentrifugalkräften notwendigen hohen Umdrehungszahlen aufwändig und teuer. Bei grossen Volumen, also in der grosstechnischen Anwendung, steigt der Aufwand, weil diese Volumen in der rotierenden Trommel zur Verfügung gestellt werden müssen, wodurch die zu bewegende Masse erheblich vergrössert wird. Grosse Zentrifugen sind aber teuer in der Anschaffung und im Unterhalt, benötigen viel Energie und bedingen sicherheitstechnische Massnahmen. Zudem kann mittels Zentrifugentechnik häufig maximal eine theoretische Trennstufe verwirklicht werden. Bei ATPS-Prozessen zur Reinigung und Isolierung von biologischem Material sind aber mitunter mehrere Trennstufen erforderlich. Die Vorteile der Extraktion mittels ATPS werden so - insbesondere im grosstechnischen Betrieb - durch den erforderlichen Einsatz der Zentrifugentechnik deutlich abgewertet.

Bei der Extraktion von Stoffen aus einer ersten Flüssigphase in eine zweite Flüssigphase ist es im Weiteren bekannt, die Phasengrenzfläche zwischen den beiden Flüssigphasen in einer porösen Membran zu immobilisieren. Dies geschieht in der Regel dadurch, dass eine der beiden Flüssigphasen die Membran benetzt und in deren Poren eindringen kann, während die andere Flüssigphase die Membran nicht benetzt bzw. von der erstgenannten Flüssigphase, die zuerst in die Membranporen eindringen konnte, am Eintritt gehindert wird (vergleiche T. Melin, R. Rautenbach, Membranverfahren, Springer-Verlag, Berlin, 2003). Die entsprechende Technologie der membrangestützten Flüssig-Flüssig-Extraktion (auch u.a. unter den Bezeichnungen "Pertraction" oder "dispersion-free solvent extraction" bekannt) steht dabei prinzipiell in direkter Konkurrenz zu herkömmlichen Extraktionstechniken, wie der Extraktion in Kolonnen (mit und ohne bewegte Einbauten), mit Mixer-Settlern und Zentrifugalextraktoren.

Durch die Immobilisierung der Phasengrenzfläche in den Membranporen ergeben sich im Prinzip folgende vorteilhafte Eigenschaften:
- Ein Dispergieren der ersten Phase in der zweiten Phase ist nicht notwendig, beide Phasen bleiben homogen. Eine Phasentrennung nach der Extraktion ist deshalb nicht erforderlich (dispersionsfreie Extraktion).
- Die Gefahr der Bildung stabiler Emulsionen ist weitestgehend eingeschränkt.
- Es ist keine Dichtedifferenz zwischen den Flüssigphasen notwendig.
- Ein kontinuierlicher Betrieb im Gegenstrom ist möglich. Dadurch können auch mehrere theoretische Trennstufen in einem Membranmodul erzielt werden.
- Die Phasenverhältnisse können in weiten Bereichen frei gewählt werden.
- Die Temperatur kann in den einzelnen Phasen unterschiedlich sein.
- Durch die Membran können grosse spezifische Austauschflächen zur Verfügung gestellt werden, insbesondere bei der Verwendung von Kapillar- und Hohlfasermodulen ("Membrankontaktoren", bis zu 4000 m²/m³ Apparatevolumen, vergleiche T. Melin, R. Rautenbach, Membranverfahren, Springer-Verlag, Berlin, 2003).
- Solange sich die Benetzungseigenschaften beider Flüssigkeiten deutlich voneinander unterscheiden, ist auch eine Extraktion ausserhalb des Zweiphasengebietes des Stoffsystems möglich.
- Der Scale-up ist einfach (ähnlich wie bei anderen Membranverfahren, z.B. bei der Ultrafiltration).

Anwendungen der membrangestützten Flüssig-Flüssig-Extraktion basierten bisher vorwiegend auf organischen Lösungsmittel / Wasser Systemen. In der Literatur ist die (mögliche) Anwendung der membrangestützten Flüssig-Flüssig-Extraktion derzeit hauptsächlich im Zusammenhang mit der Reinigung von Abwasserströmen (C. Yun et al., Membrane Solvent Extraction Removal of Priority Organic Pollutants from Aqueous Waste Streams; Ind. Eng. Chem. Res.; 1992; 31 (7); 1709-1717), der Caprolactamherstellung (W. Riedl, Membrangestützte Flüssig-Flüssig-Extraktion bei der Caprolactamherstellung, Shaker Verlag, Aachen, 2003) und der prozessintegrierten Extraktion von einfachen Produkten aus der Fermentation (G. Frank, K. Sirkar, Alcohol production by yeast fermentation and membrane extraction, Biotech. and Bioeng. Symposium, 1986, 621-631) zu finden.

Ein Verfahren zur membrangestützten Flüssig-Flüssig-Extraktion in einem organischen Lösungsmittel / Wasser System ist beispielsweise auch aus der EP 0 246 065 A1 (Celanese Corp.) bekannt. Diese Druckschrift betrifft ein Verfahren zur flüssig-flüssig-Extraktion unter Zuhilfenahme mikroporöser Membranen, bei welchem ein erhöhter Stoffdurchgang erreicht werden soll. Dazu wird die Membran zwischen den nicht miteinander mischbaren Flüssigkeiten angeordnet und durch diejenige der Flüssigkeiten benetzt, in welcher der zu extrahierende Stoff besser löslich ist; dies kann der Ausgangsstoff oder - bevorzugt - das Extraktionsmittel sein. Um die Trennfläche zwischen den Flüssigkeiten an der Membran zu stabilisieren, wird auf die nicht-benetzende Flüssigkeit ein höherer Druck ausgeübt als auf die benetzende Flüssigkeit. Bei der Planung eines Extraktionsvorgangs wird zunächst eine mit der Ausgangslösung nicht mischbare Extraktionslösung so ausgewählt, dass sich die Löslichkeit des zu extrahierenden Stoffs in den beiden Lösungen stark unterscheidet. In der Folge wird eine mikroporöse Membran ausgewählt, welche durch diejenige der Flüssigkeiten benetzt wird, in welcher der Stoff besser löslich ist. Die aufgeführten Zweiphasen-Systeme weisen allesamt eine wässrige und eine organische Phase auf: wässrige Lösung/Acetat, wässrige Lösung/Methyl-Amyl-Keton, wässrige Salzsäurelösung/Diethylen-Glykol-Dibutylether (DGDE), DGDE/KCN-Lösung.

Da derzeit die auf dem Markt erhältlichen und für die membrangestützte Extraktion geeigneten Membranen mehrheitlich hydrophoben Charakter aufweisen, ist bei konventionellen Extraktionen das organische Lösungsmittel die benetzende Flüssigkeit und Wasser die nicht benetzende Flüssigkeit.

Organische Lösungsmittel kommen allerdings für die Extraktion vieler biologischer Stoffe wegen ihrer denaturierenden Wirkung nicht in Frage.

Es gab daher Versuche, biologisches Material membrangestützt im Rahmen von wässrigen Zwei-Phasen-Systemen zu extrahieren. Werden - wie allgemein üblich - hier aber hydrophobe Membranen eingesetzt, so benetzt keine der beiden wässrigen Phasen die Membran.

Dahuron und Cussler beschreiben derartige Versuche der Extraktion von Proteinen aus wässrigen Zwei-Phasen-Systemen mittels hydrophober, mikroporöser Membranen (Protein Extraction with Hollow Fibers, AlChE Journal (34/1), 1988). Da keine der beiden wässrigen Phasen die dort verwendeten hydrophoben Membranen benetzt, wurde durch Anlegen von unterschiedlichen äusseren Drücken auf die beiden Flüssigkeiten versucht, den Flüssigphasenkontakt über die Membran herzustellen. Dabei traten aber Probleme bezüglich der Prozessstabilität auf; eine Extraktion von Cytochrome-e, Myoglobin, α-Chymotrypsin, Catalase und Urease in einem PEG / Phosphatpuffer System wurde nur durch eine äusserst präzise Einstellung und Kontrolle des Druckes zwischen Membranmodulein- und ausgang in den beiden Flüssigphasen überhaupt erst möglich. Ein (gross-)technisch sicherer und zudem einfach handhabbarer (Langzeit-)Betrieb ist mit der dort beschriebenen Technik daher nicht zu erwarten.

### Darstellung der Erfindung

Aufgabe der Erfindung ist es, ein dem eingangs genannten technischen Gebiet zugehörendes Verfahren zur Extraktion von Proteinen oder Peptiden zu schaffen, welches einfach handhabbar und in grosstechnischem Massstab einsetzbar ist.

Die Lösung der Aufgabe ist durch die Merkmale des Anspruchs 1 definiert. Gemäss der Erfindung wird mindestens eine der wässrigen Lösungen durch Zugabe eines biokompatiblen und oberflächenaktiven Stoffs so modifiziert, dass sie im Bezug auf die verwendete Membran eine unterschiedliche Benetzbarkeit gegenüber der anderen wässrigen Lösung aufweist.

Die erste wässrige Lösung mit dem zu extrahierenden biologischen Material, also den Proteinen und/oder Peptiden, steht also mit der einen Oberfläche der Membran in Kontakt, die zweite wässrige Lösung (die Reinigungsflüssigkeit) mit der anderen Oberfläche der Membran. Die Lösungen stehen zwar nicht direkt strömungstechnisch miteinander in Verbindung, das biologische Material kann aber von der einen Seite der Oberfläche auf die andere Seite übergehen.

Die Modifikation einer der wässrigen Lösungen durch Zugeben des oberflächenaktiven Stoffs führt zu einer Veränderung der Oberflächenspannung der modifizierten Lösung und damit zu einem deutlichen Unterschied der Benetzung der beiden Lösungen auf der verwendeten Membran. Die aus dem Stand der Technik bekannten Probleme und Instabilitäten, beispielsweise bezüglich des Drucks der beiden Phasen, sind also nicht zu erwarten.

Aufgrund der Biokompatibilität des verwendeten Stoffs findet keine Denaturierung des in der modifizierten wässrigen Lösung enthaltenen oder dahin extrahierten biologischen Materials statt. Die Auswahl der zu verwendenden Stoffe und deren Konzentration erfolgt abhängig von den in der ersten wässrigen Lösung vorhandenen Wertstoffen, also sowohl vom zu extrahierenden Stoff als auch von weiterem biologischem Material, welches beispielsweise in weiteren Verfahrensschritten aus der ersten wässrigen Lösung extrahiert werden soll. Wichtig ist, dass keiner dieser Wertstoffe durch den verwendeten oberflächenaktiven Stoff eine Denaturierung erfährt.

In der Tat hat sich anlässlich von Versuchen überraschend herausgestellt, dass durch die Zugabe des Stoffs die Membranextraktion von biologischem Material aus einer ersten wässrigen Lösung in eine zweite wässrige Lösung auf einfache und effiziente Weise ermöglicht wird. Mit dem hier vorgestellten Verfahren wird es deshalb erstmals ermöglicht, unterschiedliches biologisches Material mittels unterschiedlicher wässriger Zwei-Phasen-Systeme mit Membrankontaktoren zuverlässig zu extrahieren. Dabei müssen sowohl an die verwendeten Membrankontaktoren als auch an die für die Extraktion notwendigen Betriebsparameter keine besonderen Anforderungen gestellt werden. Somit können sowohl die Vorteile des wässrigen Zwei-Phasen-Systems als auch die Vorteile der Membranextraktion ausgenutzt werden.

Überraschenderweise hat sich anlässlich der Versuche weiter gezeigt, dass die Zugabe des oberflächenaktiven Stoffs zu der einen wässrigen Lösung zusätzlich den Vorteil bieten kann, dass die Verteilung des biologischen Materials gegenüber Systemen ohne oberflächenaktive Komponente positiv zugunsten einer höheren Konzentration in der Aufnehmerphase beeinflusst wird, was zu einem erhöhten Extraktionsgrad und/oder einer Verringerung der für die Extraktion notwendigen Trennstufen beiträgt. Stehen für eine Extraktion mehrere mögliche oberflächenaktive Stoffe zur Verfügung, kann gezielt derjenige ausgewählt werden, welcher eine bestmögliche Beeinflussung bewirkt.

Vorzugsweise wird die mindestens eine der wässrigen Lösungen so modifiziert, dass sie die verwendete poröse Membran benetzt und in deren Poren eindringen kann, während die andere wässrige Lösung die Membran nicht benetzt und somit nicht in die Poren der Membran eindringt.

Der Kontaktwinkel zwischen einer Flüssigkeit und der Membranoberfläche kann auf einfache Weise bestimmt werden und liefert ein Mass für die Benetzbarkeit. Eine Flüssigkeit mit einem Kontaktwinkel zur Membran (unabhängig ob hydrophob oder hydrophil) von mindestens 90° gilt als nicht benetzend, eine andere Flüssigkeit mit einem Kontaktwinkel von weniger als 90° gilt als benetzend. Durch die erfindungsgemässe Modifikation vor allem der einen Phase wird also sichergestellt, dass die modifizierte Phase die Membran vorrangig benetzt. Die Modifikation hat auch zur Folge, dass eine der Lösungen vor der anderen wässrigen Lösung in die Poren der Membran eindringen kann. Weil der Kontaktwinkel einfach und damit schnell und ohne grossen Aufwand messbar ist, kann zur Vorbereitung einer Extraktion durch eine kurze Versuchsreihe der Effekt unterschiedlicher oberflächenaktiver Stoffe mit unterschiedlichen Konzentrationen untersucht werden. Die Zugabe dieses Stoffes für die Extraktion kann dann, basierend auf diesen Vorversuchen, in notwendiger und hinreichender Konzentration erfolgen.

Bevorzugt wird die eine der wässrigen Lösungen derart modifiziert, dass eine Differenz zwischen einem ersten Kontaktwinkel zwischen der ersten wässrigen Lösung und der Oberfläche der Membran und einem zweiten Kontaktwinkel zwischen der zweiten wässrigen Lösung und der Oberfläche der Membran mindestens 5°, bevorzugt mindestens 10°, beträgt. Die problemlose Durchführung der Extraktion wird durch den Unterschied in der Benetzbarkeit der beiden wässrigen Phasen ermöglicht, wofür die Differenz der Kontaktwinkel ein Mass liefert.

Mit Vorteil ist die poröse Membran hydrophob. Derartige Membranen sind zuverlässig kommerziell verfügbar.

Bei einer hydrophoben Membran wird die eine der wässrigen Lösungen bevorzugt derart modifiziert, dass ein Kontaktwinkel zwischen der modifizierten wässrigen Lösung und einer Oberfläche der hydrophoben Membran kleiner ist als 90°, bevorzugt kleiner als 50°.

Bevorzugt ist der zugegebene oberflächenaktive Stoff ein Tensid. Die durchgeführten Versuche haben gezeigt, dass sich durch Zugabe eines biokompatiblen Tensids die eine der wässrigen Lösungen derart modifizieren lässt, dass eine problemlose membrangestützte Extraktion ermöglicht wird.

Dies ist deshalb überraschend, weil in der einschlägigen Fachliteratur (T. Melin, R. Rautenbach, Membranverfahren, Springer-Verlag, Berlin, 2003) die Gegenwart von Tensiden als kritisch für die Prozessstabilität der membrangestützten Flüssig-Flüssig-Extraktion bewertet wurde, da sich diese auf den Membranoberflächen anreichern würden und so die unterschiedliche Benetzbarkeit der beiden Flüssigphasen aufheben könnten. Dies führe in Extremfällen zum Phasendurchbruch (an anderer Stelle wird auch von "Fehlphasenbildung " gesprochen), bei dem eine der beiden Phasen in die andere übertrete.

Anlässlich der durchgeführten Versuchsreihen konnten aber während ungefähr fünf Monaten mit demselben Membrankontaktor wiederholt Extraktionen unter der Zugabe von Tensiden durchgeführt werden, ohne dass je eine Fehlphasenbildung oder eine instabile Prozessfahrweise beobachtet wurde. Auch konnte der Kontaktor mit unterschiedlichen wässrigen Zwei-Phasen-Systemen, unterschiedlichen Proteinen und abwechselnd sowohl mit als auch (in Vergleichsversuchen) ohne Tensid betrieben werden. Die Ergebnisse von Wiederholungsversuchen waren stets reproduzierbar und die Prozessstabilität war immer gegeben.

Der Membrankontaktor wurde nach Ende eines Versuchs lediglich jeweils mit heissem Wasser gespült, wie dies bei Membranverfahren (z.B. der Ultrafiltration) üblich, oft sogar ohnehin zwingend erforderlich ist.

Extraktions- und/oder Benetzungsversuche wurden mit den Tensiden Tween@ 20 (CAS 9005-64-5), Triton® X-1 00 (CAS 9002-93-1) und Brij® 35 P (CAS 9002-92-0) durchgeführt. Die Ergebnisse haben bestätigt, dass diese drei unterschiedlichen Tenside im erfindungsgemässen Verfahren zur Modifikation der wässrigen Phasen erfolgreich einsetzbar sind. Dies deutet darauf hin, dass auch andere biokompatible Tenside im Rahmen der Erfindung verwendbar sind.

Mit Vorteil wird die eine der wässrigen Lösungen derart modifiziert, dass eine Konzentration des Tensids in der modifizierten wässrigen Lösung höchstens 2.5 Gew.%, bevorzugt weniger als 1.0 Gew.%, beträgt. Bereits eine derartig geringe Konzentration reicht aus, um die geforderte unterschiedliche Benetzung der beiden Flüssigphasen auf der Membran herbeizuführen. Gleichzeitig besteht bei derartig niedrigen Konzentrationen kaum die Gefahr der Akkumulation dieser Tenside in und auf der Membranmatrix und eine Schädigung der Wertstoffe durch das Tensid kann wirksam verhindert werden.

Bei Verwendung einer hydrophilen Membran für die Trennaufgabe wird mit Vorteil die eine der beiden wässrigen Lösungen derart modifiziert, dass ein Kontaktwinkel zwischen der modifizierten wässrigen Lösung und einer Oberfläche der hydrophilen Membran mindestens 90° beträgt.

Vorzugsweise wird als Stoff zum Modifizieren der einen wässrigen Lösung ein Stoff ausgewählt, welcher sich verglichen mit der anderen wässrigen Lösung stark bevorzugt in der zu modifizierenden wässrigen Lösung anreichert. Diese Selektivität führt dazu, dass im Wesentlichen die Benetzbarkeit der zu modifizierenden Lösung beeinflusst wird. Die Eigenschaften der anderen Lösung verändern sich auch dann deutlich weniger, wenn die andere Lösung mit dem oberflächenaktiven Stoff in Kontakt kommt. Entsprechend kann auf einfache Weise ein grosser Unterschied der Benetzbarkeit der Lösungen erreicht werden.

Bevorzugt ist die Membran eine poröse Flach-, Kapillar- oder Hohlfasermembran. Das erfindungsgemässe Verfahren lässt sich mit derartigen erprobten und einfach verfügbaren Membranen auf effiziente Weise durchführen.

Ein maximaler Porendurchmesser der Membran beträgt 2 µm. Die Membran weist also eine mikroporöse Struktur auf, derart, dass sie durchlässig für das zu extrahierende biologische Material ist. Je nach zu extrahierendem Wertstoff (biologischem Material) sind auch deutlich geringere Durchmesser bis hinunter zu ca. 0.01 µm und weniger einsetzbar. Bei Porendurchmessern von mehr als 2 µm ist ein stabiler Extraktionsvorgang nicht mehr möglich, weil die Durchgänge derart gross sind, dass die Lösungen die Poren passieren können, ohne dass sie von der Membran wesentlich beeinflusst werden. Die Phasengrenzfläche kann aus diesem Grund nicht mehr in der Membranpore immobilisiert werden, und die Phasen bleiben nicht mehr homogen.

Die Membran kann aus einem organischen, anorganischen oder Composite-Material gefertigt sein.

Bevorzugt enthält eine der wässrigen Lösungen ein Polymer, insbesondere PEG (Polyethylenglykol) oder Dextran. In der anderen wässrigen Lösung ist vorrangig ein Salz, z. B.

Natriumsulfat (Na₂SO₄) oder ein Phosphatpuffer, z. B. ein Kaliumhydrogenphosphat (K₂HPO₄ / KH₂PO₄), gelöst.

Bei anderen wässrigen Zwei-Phasen-Systemen, welche im Rahmen der Erfindung einsetzbar sind, enthält die erste wässrige Lösung ein erstes Polymer und die zweite wässrige Lösung ein zweites Polymer, wobei die Polymere wasserlöslich und nicht oder nur geringfügig miteinander mischbar sind. Die zwei Polymere sind bevorzugt PEG und Dextran.

Die Erfindung ist aber nicht auf die angegebenen wässrigen Zwei-Phasen-Systeme beschränkt. Die Ergebnisse der durchgeführten Versuche deuten darauf hin, dass die membrangestützte Flüssig-Flüssig-Extraktion auch mit anderen bereits bekannten oder noch zu entwickelnden wässrigen Zwei-Phasen-Systemen betriebssicher und langzeitstabil anwendbar ist.

Mit Vorteil wird mindestens eine der wässrigen Lösungen vor oder nach der Extraktion destillativ, mittels Membranverfahren, Flockung und/oder durch andere geeignete Trennverfahren in ihren physikalischen, chemischen und/oder thermodynamischen Eigenschaften verändert. Derartige, an sich bekannte Massnahmen können die Extraktionsleistung oder die Qualität des extrahierten Stoffs verbessern.

Im Rahmen der Erfindung wird ein biokompatibler Stoff in dem erfindungsgemäßen Verfahren zur Extraktion von biologischem Material aus einer ersten wässrigen Lösung in eine zweite wässrige Lösung verwendet, wobei zwischen den wässrigen Lösungen eine poröse Membran angeordnet ist und wobei mindestens eine der wässrigen Lösungen durch Zugabe des Stoffs so modifiziert wird, dass sie im Bezug auf die verwendete Membran eine gegenüber der anderen wässrigen Lösung unterschiedliche Benetzbarkeit aufweist.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine schematische Darstellung des Prinzips der membrangestützten Extraktion;
- Fig. 2A,B: grafische Darstellungen der Konzentration der im Rahmen von Versuchen extrahierten Proteine in den beiden Phasen eines wässrigen Zwei-Phasen-Systems;
- Fig. 3: eine schematische Darstellung des Kontaktwinkels von benetzenden und nicht benetzenden Flüssigkeiten auf einer Oberfläche;
- Fig. 4: eine schematische Darstellung der für die Membranextraktionsversuche eingesetzten Anlage;
- Fig. 5: eine schematische Darstellung des verwendeten Membrankontaktors;
- Fig. 6: eine grafische Darstellung des Konzentrationsverlaufs von Lysozym während eines Extraktionsversuchs mit späterer Zugabe des Tensids; und
- Fig. 7: eine schematische Darstellung einer verfahrenstechnischen Realisierung des erfindungsgemässen Verfahrens.

Grundsätzlich sind in den Figuren gleiche Teile mit gleichen Bezugszeichen versehen.

### Wege zur Ausführung der Erfindung

### Herkömmliche wässrige Zwei-Phasen-Systeme

Aus der Literatur (z. B. C. Grossmann, Untersuchungen zur Verteilung von Aminosäuren und Peptiden auf wässrige Zwei-Phasen-Systeme, Dissertations Druck Darmstadt, Kaiserslautern, 2002; J. Brenneisen, Zur Verteilung von Proteinen auf wässrige Zwei-Phasen-Systeme, Der Andere Verlag, Osnabrück, 2001; J. Rydberg et al., Principles and practices of solvent extraction, Marcel Dekker Inc., New York, 1992, S. 339-347 und H. Walter et al., Methods in Enzymology - Aqueous Two-Phase Systems, Academic Press, San Diego, 1994, Vol. 228) sind beispielsweise folgende häufig eingesetzte wässrige Zwei-Phasen-Systeme (ATPS) bekannt:
Wasser / PEG 6000 / Dextran 500,
Wasser / PEG 6000 / K₂HPO₄,
Wasser / PEG 6000 / Dextran 70 / Na₂SO₄,
Wasser / Dextran 48 / PEG 4000,
Wasser / Dextran 48 / PEG 4000 / Phosphatpuffer / NaCl,
Wasser / PEG 4000 / (K₂HPO₄ / KH₂PO₄).

Die Herstellung eines wässrigen Zwei-Phasen-Systems erfolgt durch das Mischen wässriger Lösungen der angegebenen Polymere bzw. Salze. Nach dem Mischen bilden sich zwei Phasen aus. Da bei den ATPS geringere Dichteunterschiede und grössere Viskositäten als bei der herkömmlichen Flüssig-Flüssig-Extraktion auftreten, dauert die Phasentrennung im Schwerefeld länger als bei organischen Lösungsmittel/Wasser-Systemen. Gemäss der erwähnten Druckschrift Rydberg et al. ist hierbei mit Zeiten zwischen fünf und dreissig Minuten zu rechnen.

Weil der Dichteunterschied der beiden Phasen aufgrund des hohen Wasseranteils gering ist (gewöhnlich weniger als 60 kg/m³), erfolgt die vollständige Trennung der Phasen anschliessend durch Zentrifugation. Diese verteuert das Verfahren erheblich und nimmt eine erhebliche Zeit in Anspruch (Grössenordnung dreissig Minuten), wodurch der Durchsatz bei konventionellen Extraktionen erheblich vermindert wird.

### Membrangestützte Extraktion

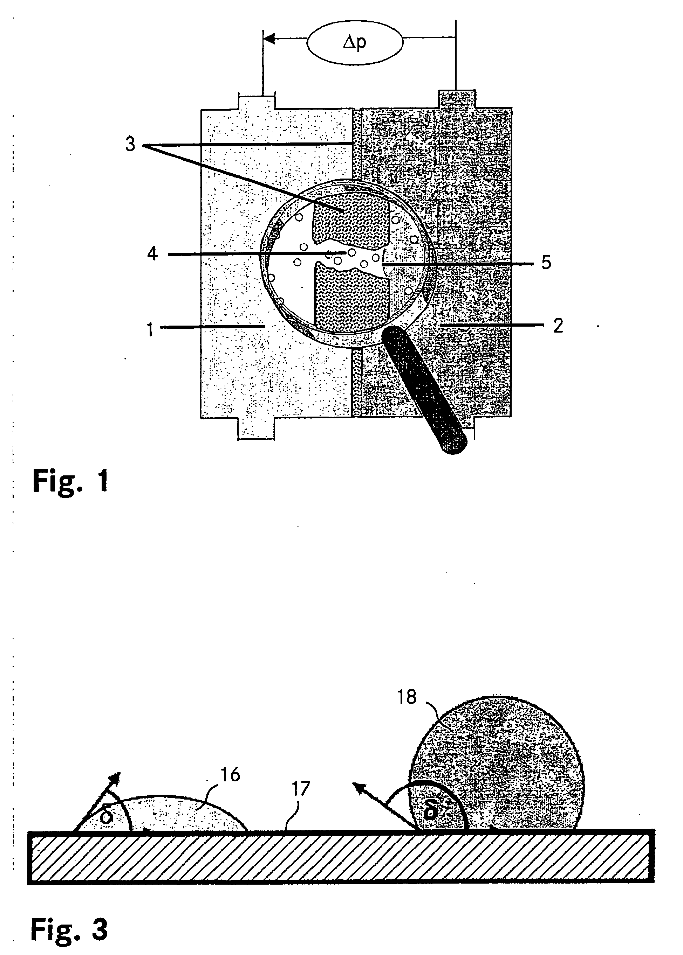

Das Prinzip der membrangestützten Extraktion ist in der Figur 1 dargestellt. Die beiden Flüssigphasen 1, 2 werden mittels einer porösen Membran 3 voneinander getrennt, müssen also nicht, wie bei herkömmlichen Flüssig-Flüssig-Extraktionen, intensiv miteinander vermischt werden. Das Dispergieren einer Phase in der anderen Flüssigphase ist also nicht notwendig, man spricht deshalb auch von dispersonsfreier Flüssig-Flüssig-Extraktion. So ist eine Phasentrennung (im Schwer- oder Zentrifugalkraftfeld) nach der Extraktion nicht erforderlich.

Die Membran 3 wird dabei überwiegend von der ersten Phase 1 benetzt. Da die Membranporen 4 einen kleinen Durchmesser von weniger als ca. 2 µm aufweisen und die Wandstärken der Membran mit weniger als ca. 300 µm gering sind, werden die Membranporen 4 aufgrund der Kapillarität vollständig mit der benetzenden Phase 1 gefüllt. Deshalb bildet sich die Phasengrenzfläche 5 zwischen den beiden Flüssigphasen 1, 2 an den Porenausgängen der Membran 3 auf derjenigen Seite der Membran 3 aus, an welcher die nicht benetzende Flüssigphase 2 anliegt.

Um zu verhindern, dass die benetzende Phase 1 in die nicht benetzende Phase 2 eintritt ("Ausbluten"), kann letztere unter einen gewissen Druck gesetzt werden, so dass zwischen den beiden Flüssigphasen 1, 2 ein transmembraner Druck Δp herrscht. In der Regel sind hierfür Drücke von weniger als 400 mbar ausreichend (K. Wecker, Untersuchungen zum Trennverhalten eines Hohlfasermoduls bei der Flüssig-Flüssig-Extraktion und Vergleich mit der Leistung einer Packungskolonne, Dissertation, Universität Erlangen-Nürnberg, Erlangen, 1998). Mit dem transmembranen Druck wird gleichzeitig sichergestellt, dass die Phasengrenzfläche 5 in der Membranpore 4 immobilisiert ist.

Gemäss T. Melin, R. Rautenbach, Membranverfahren, Springer-Verlag, Berlin, 2003 und W. Riedl, Membrangestützte Flüssig-Flüssig-Extraktion bei der Caprolactamherstellung, Shaker Verlag, Aachen, 2003, besitzt die Membran bei der membrangestützten Flüssig-Flüssig-Extraktion gegenüber den zu extrahierenden Komponenten nahezu keine Selektivität. Dies ist dadurch begründet, dass die Porendurchmesser üblicherweise deutlich grösser sind als die Moleküldurchmesser der gelösten Komponenten. Dies ergibt sich beispielsweise aus der folgenden Tabelle, in welcher Moleküldurchmesser von ausgewählten, gelösten Komponenten im Vergleich mit einer 0,1 µm-Membranpore aufgeführt sind:

| in Wasser gelöster Stoff | Durchmesser d [µm] | d_{Pore} (0,1 µm) / d |
|---|---|---|
| Glucose | 4,44×10⁻⁴ | ~ 225 : 1 |
| Caprolactam | < 6×10⁻⁴ | ~ 160 : 1 |
| β-Dextrin | 8,00×10⁻⁴ | ~ 125 : 1 |

Wie die Tabelle zeigt, beträgt das Verhältnis des Membran-Porendurchmessers (0,1µm) zum Durchmesser der dargestellten Moleküle mindestens 125 : 1.

### Durchgeführte Versuche

Für die Durchführung einer ersten, ausführlichen Versuchsreihe wurde ein wässriges Zwei-Phasen-System bestehend aus Polyethylenglykol 4'000/Phosphatpuffer/Wasser mit einem pH-Wert von 7.2 gewählt. Zur Herstellung des Systems wurden 2000g PEG-Stammlösung aus 500 g PEG und 1500 g deionisiertem Wasser und 1800g Puffer-Stammlösung aus 220 g Kaliumdihydrogenphosphat (KH₂PO₄) und 280 g di-Kaliumhydrogenphosphat (K₂HPO₄) und 1300 g deionisiertem Wasser in einen Scheidetrichter eingewogen und gemischt. Nachdem sich die Phasen getrennt hatten, wurden sie bei Raumtemperatur (22 °C) separiert. Beide noch leicht trüben Phasen konnten durch Zugabe von jeweils 250g deionisiertem Wasser geklärt werden.

Zunächst wurden konventionelle Extraktionsversuche im Scheidetrichterverfahren durchgeführt. Dabei wurden die Gleichgewichtsverteilungen der Proteine Bovine Serum Albumin (BSA, Molekularmasse 67 kDa) und Lysozym (Molekularmasse 14.6 kDa) auf das wässrige Zwei-Phasen-System bei Raumtemperatur und bei 36 °C vermessen.

Bei der Messung der Verteilung von BSA wurden drei unterschiedliche BSA-Konzentrationen (1000 mg/System, 1500mg/System, 2000mg/System) in einem ATPS untersucht. Dazu wurde dem hergestellten ATPS BSA in kristalliner Form zugegeben und durch leichtes Schütteln gelöst. Anschliessend wurde das System drei Minuten im Scheidetrichter geschüttelt. Die Phasen trennten sich bereits nach wenigen Minuten, deutliche Trübungen in beiden Phasen waren aber noch vorhanden. Das System wurde über Nacht zum Abscheiden stehen gelassen und war am nächsten Morgen (nach ca. 14h) klar. Die Phasen wurden bei einer Raumtemperatur von 23 °C separiert und deren Volumen gemessen. Die hierbei wieder leicht trüb gewordenen unteren Phasen wurden dreissig Minuten bei 4800 U/min (r=10 cm) zentrifugiert. Nach dem Zentrifugieren waren die Phasen immer noch leicht trüb, jedoch bildete sich eine dünne obere Phase aus. Mit einer Pipette wurde der untere Teil abgesogen. Ein Teil dieser Probe wurde unter dem Lichtmikroskop bei 40-facher Vergrösserung betrachtet. Es konnte deutlich festgestellt werden, dass die Trübung von Bläschen der zweiten Phase stammt. Der Bläschendurchmesser lag bei 2.5-9 µm. Die Proben wurden wiederum über Nacht stehen gelassen. Am nächsten Tag waren auch diese Proben klar, auf der Oberfläche konnte wiederum jeweils ein dünner Film der oberen Phase festgestellt werden.

Der BSA-Gehalt der klaren Proben wurden photospektrometrisch bei 280 nm im Doppel bestimmt. Da die BSA-Konzentrationen in den PEG-Phasen unterhalb des Messbereichs lagen, wurden diese über die Massenbilanz errechnet.

Parallel dazu wurden dieselben Versuche durchgeführt mit einem ATPS, welchem 0.55 g des nichtionischen Tensids Tween@ 20 (Polyethylenglycolsorbitan monolaurat) beigemischt wurde. Mit blossem Auge konnte zunächst festgestellt werden, dass die Systeme mit Tween® 20 wesentlich mehr Schaum bilden als diejenigen ohne das Tensid.

Zur Untersuchung der Verteilung von Lysozym auf das ATPS wurden wiederum unterschiedliche Lysozym-Konzentrationen (250 mg/System, 500 mg/System, 750 mg/System) in einem ATPS ohne Tween® 20 und einem ATPS mit 0.55 g beigemischtem Tween@ 20 untersucht. Lysozym kann nicht wie BSA in kristalliner Form in das ATPS gegeben werden, da es Agglomerate bildet, welche sich nur schwer lösen. Um dies zu umgehen, wurde eine 9.7%ige Lysozymlösung in Wasser hergestellt. Diese Lösung ersetzte einen Teil (je nach benötigter Proteinmenge 2.5g, 5g, 7.5g) der 10.0g Wasser, die bei der Herstellung des ATPS benötigt werden. Das restliche zur Herstellung des ATPS erforderliche Wasser wurde nach der Zugabe der Lysozymlösung beigemischt.

Das weitere Vorgehen entsprach dem der Systeme mit BSA. Auch das Entmischungsverhalten war identisch. Es wurden beide Phasen zentrifugiert und analysiert.

Ein weiteres System mit 60mg Lysozym wurde zusätzlich bei 36 °C vermessen, hierzu wurde das hergestellte und mit Protein versetzte ATPS im Heizbad auf 36 °C temperiert, danach drei Minuten geschüttelt und über Nacht bei 36 °C zum Entmischen ruhen gelassen. Die Phasen wurden separiert und durch Doppelbestimmung wurde der Gehalt an Lysozym analysiert.

Die folgenden Tabellen zeigen eine Zusammenfassung der Analyseergebnisse. Angegeben ist jeweils die Einwaage des entsprechenden Proteins, ob das Tensid Tween@ 20 zugegeben worden ist oder nicht, sowie die Konzentrationen c in der oberen Phase (Top) sowie in der unteren Phase (Bottom) der beiden Flüssigphasen im Scheidetrichter.

**Bovine Serum Albumin (BSA) bei Raumtemperatur (23 °C):**

| Einwaage (mg) | Tween@ 20 | cBSA, Top (mg/ml) | cBSA, Bottom (mg/ml) | cTop / cBottom |
|---|---|---|---|---|
| 1000.6 | nein | 2.476 | 20.884 | 1:8.4 |
| 1496.7 | nein | 1.538 | 32.132 | 1:20.9 |
| 2013.3 | nein | 2.059 | 42.755 | 1:20.8 |
| 998.6 | ja | 0.661 | 22.192 | 1:33.6 |
| 1498.1 | ja | 0.762 | 32.724 | 1:42.9 |
| 2016.4 | ja | 1.510 | 43.188 | 1:28.6 |

**Lysozym bei Raumtemperatur (24 °C):**

| Einwaage (mg) | Tween® 20 | cLYS,Top (mg/ml) | cLYS, Bottom (mg/ml) | cTop / cBottom |
|---|---|---|---|---|
| 244.0 | nein | 1.2254 | 4.7888 | 1:3.9 |
| 488.7 | nein | 2.2300 | 8.5201 | 1:3.8 |
| 735.1 | nein | 3.0945 | 14.5150 | 1:4.7 |
| 247.3 | ja | 1.1847 | 4.9058 | 1:4.1 |
| 490.1 | ja | 2.0446 | 9.6627 | 1:4.7 |
| 750.0 | ja | 2.8467 | 15.0180 | 1:5.3 |

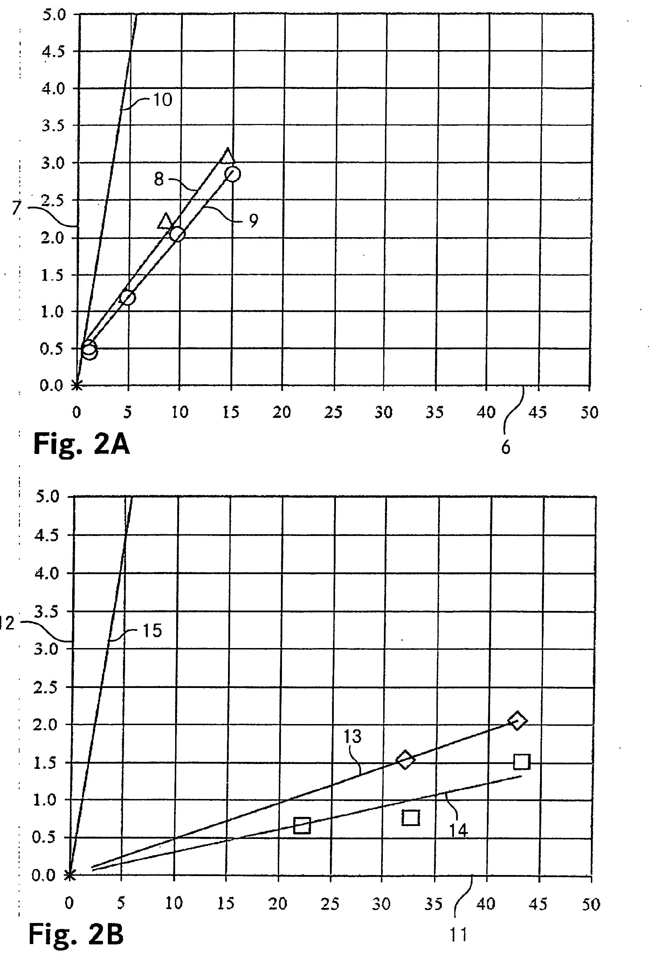

Die Resultate sind in den Figuren 2A, 2B grafisch dargestellt. Die Figur 2A zeigt die Verteilung von Lysozym in den beiden Phasen des ATPS. Entlang der horizontalen Achse 6 sind die oben angegebenen Konzentrationen in der Puffer-Phase in mg/ml angegeben, entlang der vertikalen Achse 7 in denselben Einheiten die angegebene Konzentration in der PEG-Phase. Die Linie 8 verbindet diejenigen Resultate, welche ohne Zugabe des Tensids gemessen worden sind, die Linie 9 verbindet diejenigen Messpunkte, welche die gemessenen Konzentrationen mit Zugabe des Tensids angeben. Die Gerade 10 gibt zu Vergleichszwecken den Fall einer gleichen Konzentration in beiden Phasen an.

Auf ähnliche Weise zeigt die Figur 2B die Verteilung von BSA in den beiden Phasen. Entlang der horizontalen Achse 11 ist wiederum die Konzentration in der Puffer-Phase, entlang der vertikalen Achse 12 die Konzentration in der PEG-Phase angegeben, beide in mg/ml. Die Messpunkte ohne Tensidzugabe sind durch die Linie 13 verbunden, diejenigen mit Zugabe des Tensids durch die Linie 14. Die Gerade 15, welche einer gleichen Konzentration in beiden Phasen entspricht, dient wiederum zu Vergleichszwecken.

Wie aus beiden Figuren ersichtlich ist, verteilen sich sowohl BSA als auch Lysozym bevorzugt auf die untere, pufferreiche Phase. Durch Zugabe von Tween® 20 wird die Verteilung bei beiden Proteinen auf überraschende Weise noch zu Gunsten der (unteren) Puffer-Phase verschoben. Tween® 20 scheint beide Proteine zusätzlich aus der (oberen) PEG-Phase zu verdrängen, wodurch der Extraktionserfolg noch verbessert werden kann, wenn - wie hier - die Puffer-Phase als Aufnehmerphase gewählt wird.

Als Nächstes wurden Vorversuche für die Membranextraktion durchgeführt. Da die Benetzungseigenschaften der wässrigen Lösungen für einen stabilen Betrieb der membrangestützten Extraktion wesentlich sind, wurde das Benetzungsverhalten der beiden wässrigen Lösungen des ATPS untersucht. Verfahren, welche mittels geringem Aufwand schnell über die Durchführbarkeit der membrangestützten Extraktion Auskunft geben, sind die Bestimmung des Kontaktwinkels eines auf einer Fläche aus dem Material der für die Extraktion ausgewählten Membran aufgebrachten Flüssigkeitstropfens oder die Messung der kapillaren Steighöhe beim Eintauchen einer Kapillar- bzw. Hohlfasermembran in die Flüssigphase (W. Riedl, Membrangestützte Flüssig-Flüssig-Extraktion bei der Caprolactamherstellung, Shaker Verlag, Aachen, 2003).

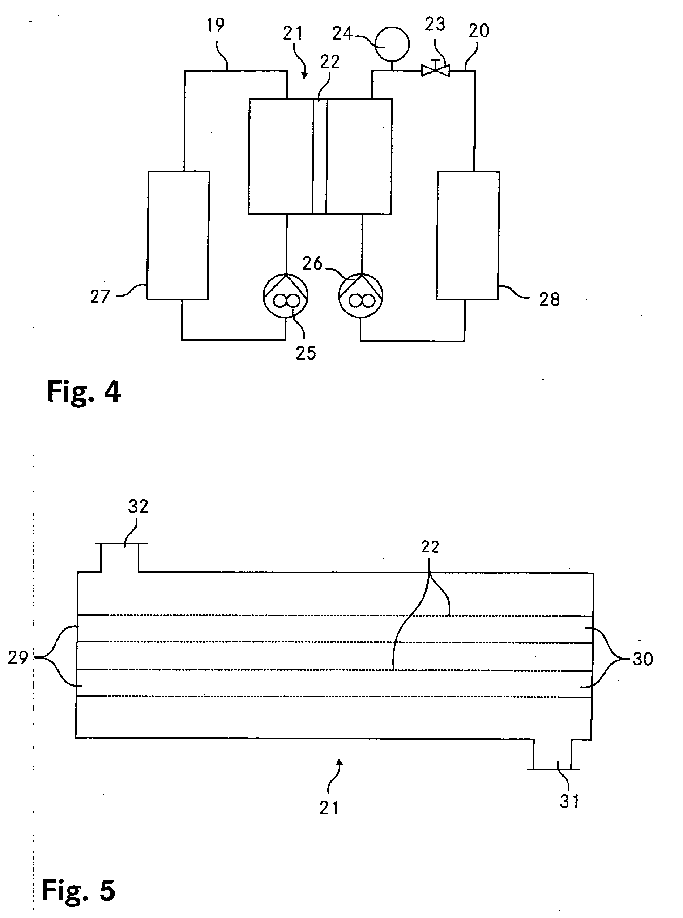

Bei Kontaktwinkeln 0° < δ ≤ 90° benetzt eine Flüssigkeit 16 die Oberfläche 17, bei Kontaktwinkeln 90° < δ < 180° gilt eine Flüssigkeit 18 als nicht benetzend (Figur 3). Da für die Durchführbarkeit der membrangestützten Extraktion vor allem ein wesentlicher Unterschied des Benetzungsverhaltens der beiden wässrigen Lösungen erforderlich ist, genügt es auch schon festzustellen, dass eine der Lösungen einen deutlichen Tropfen auf der Membran ausbildet und die andere nicht.

Die Messung der kapillaren Steighöhe beruht darauf, dass eine benetzende Flüssigkeit in einer Kapillare aufsteigt. Da sowohl die Porenlänge als auch der Porendurchmesser in Membranen sehr klein sind, kann schon bei einer geringen Steighöhe davon ausgegangen werden, dass die Membran vollständig benetzt wird.

Es wurden präzise Kontaktwinkelmessungen der beiden Phasen des ATPS durchgeführt, wobei die auf der Membranoberfläche entstehenden Tropfen der Phasen aus mehreren Richtungen fotografisch festgehalten wurden. Anhand der Fotografien mehrerer Tropfen wurde dann der Winkel bestimmt. Die Messungen haben erwartungsgemäss gezeigt, dass weder die wässrige PEG-Phase noch die wässrige Puffer-Phase die verwendete hydrophobe Membran (Celgard® 2400) aus Polypropylen benetzen. Für die Puffer-Phase wurde ein Kontaktwinkel von 102±4°, für die PEG-Phase ein Kontaktwinkel von 105±3° bestimmt. Entsprechend wurde auch keine kapillare Steighöhe festgestellt. Weiterführende Versuche bestätigten, dass durch Beimischen des nichtionischen Tensids Tween® 20 zum ATPS selektiv die PEG-Phase so verändert wird, dass sie die verwendete Membran benetzt. Die Kontaktwinkel verändern sich zu 92±4° für die Puffer-Phase und zu 47±2° für die PEG-Phase.

Tween® 20 reichert sich nämlich im Gleichgewicht stark bevorzugt in der PEG-Phase an, so dass die Benetzungseigenschaften der Puffer-Phase durch das Tensid weniger stark geändert werden als diejenigen der PEG-Phase. Durch Löslichkeitsversuche von Tween® 20 in den verwendeten wässrigen Lösungen der ATPS-Komponenten, bei denen festgestellt wurde, dass sich Tween® 20 in der PEG-Lösung löst, in der Puffer-Lösung aber eine eigene Phase bildet, konnte dies bestätigt werden. Dieses Lösungsverhalten dürfte auf der Strukturverwandtheit zwischen PEG und Tween® 20 beruhen.

Die für die Membranextraktionsversuche eingesetzte Anlage ist schematisch in der Figur 4 dargestellt und besteht aus zwei voneinander unabhängigen Kreisläufen 19, 20 für die Aufnehmer- und die Abgeberphase. Als Schnittstelle zwischen den beiden Kreisläufen 19, 20 ist ein Membrankontaktor 21 mit einer Membran 22 eingebaut. Um einen transmembranen Druck einzustellen, ist der Kreislauf 20 der die Membran 22 nichtbenetzenden Phase mit einem Regelventil 23 und einem Manometer 24 ausgerüstet. In beiden Kreisläufen 19, 20 können Temperatur, Durchfluss und Vorlagevolumen unabhängig voneinander eingestellt werden. Der Transport der Phasen durch die Kreisläufe 19, 20 wird durch jeweils eine Pumpe 25, 26 bewirkt.

Für die Versuche wurde ein kommerziell verfügbarer Membrankontaktor mit Polyproyplen-Hohlfasermembranen verwendet (der Firma Liqui-Cel®, mit Celgard® X40 Hohlfasermembranen; laut Hersteller mit vergleichbaren (Benetzungs-)Eigenschaften wie die Celgard® 2400 Flachmembranen). Er hat eine Austauschfläche von 1.4 m². Die Figur 5 zeigt schematisch und vereinfacht die Funktionsweise des Membrankontaktors 21 in einem Schnittbild. Durch den Zugang lumen-side 29 wird die eine Phase durch die Hohlfasermembran 22 geleitet, bis sie am Abgang lumen-side 30 den Membrankontaktor 21 wieder verlässt. Die durch den Zugang shell-side 31 zugeführte Phase tritt in den Membrankontaktor 21 ein und verlässt diesen über den Abgang shell-side 32.

Bei sämtlichen Versuchen wurde die PEG-Phase als Abgeberphase und die Puffer-Phase als Aufnehmerphase gewählt. Die Anlage wurde in Kreislauffahrweise betrieben, um eine analytisch besser erfassbare Konzentrationsänderung zu erreichen. Wegen der geringen Proteinlöslichkeit in der Abgeberphase (siehe oben) wurde das Volumen dieser auf ein Vielfaches des Volumens der Aufnehmerphase erhöht.

Zu Versuchsbeginn wurden die Phasen des ATPS, wie oben beschrieben, hergestellt und in die Vorlagegefässe 27, 28 der Membrananlage gegeben. Die benötigte Menge des jeweiligen Proteins wurde zuvor in dem zur Verdünnung der PEG-Phase verwendeten Wasser gelöst.

Zuerst wurde dann der Kreislauf 20 der nichtbenetzenden Phase (Aufnehmer- bzw. Puffer-Phase) an den Membrankontaktor 21 angeschlossen und eingeschaltet. Bei Bedarf wurde dann mit dem Druckregelventil 23 der gewünschte transmembrane Druck eingestellt, wobei häufig ein Druck von 40 mbar (gemessen am Kontaktorausgang) eingestellt wurde.

Als Nächstes wurde der Kreislauf 19 der benetzenden Phase an den Membrankontaktor 21 angeschlossen und in Betrieb genommen.

Während der Extraktion wurden Proben der Aufnehmerphase genommen und analysiert. Die Konzentration der Proteine wurde photospektrometrisch bestimmt. Solange die Proben für die Analyse nicht verdünnt werden mussten, wurden sie umgehend nach der Bestimmung wieder der Aufnehmerphase zugeführt. Zur Bestätigung, dass tatsächlich das Protein transferiert wurde, wurde das UV/Vis-Spektrum einer Probe mit dem des Proteins verglichen.

Um eine Kontamination der Anlage zu minimieren, wurde die Anlage nach jedem Versuch gereinigt. Die Vorlagegefässe 27, 28, die Pumpen 25, 26 und die Leitung wurden zuerst mit deionisiertem Wasser und dann mit Aceton gespült. Durch Ausblasen mit Druckluft wurde die Anlage getrocknet. Der Membrankontaktor 21 wurde mindestens eine Stunde mit heissem Wasser durchspült und danach jeweils mit Aceton und 2-Propanol ausgeschwenkt. Danach wurde der Membrankontaktor 21 während ca. einer Stunde mit Argon getrocknet.

Der Gesamtstoffdurchgangskoeffizient K wurde, wie in W. Riedl, Membrangestützte Flüssig-Flüssig-Extraktion bei der Caprolactamherstellung, Shaker Verlag, Aachen, 2003 beschrieben, aus den Messresultaten berechnet. Diese Grösse ist ein Mass für den Stofftransport durch die Membran und damit für die Extraktionsleistung und ist unabhängig vom Vorlagevolumen, der Einwaage und der installierten Membranfläche.

Um die membrangestützte Extraktion zu ermöglichen, wurde zur Benetzung der Membran durch die PEG-Phase dem ATPS Tween® 20 zugegeben. Entweder erfolgte hierbei eine Zugabe von 27.8g Tween® 20 vor dem Mischen im Scheidetrichter (Versuche 1, 2) oder direkt in die zur Membranextraktion verwendete PEG-Phase (1g Tween® 20 pro 100ml PEG-Phase, Versuche 3-7), was das Herstellungsverfahren der Lösungen vereinfacht.

Die folgende Tabelle zeigt die Ergebnisse der Membranextraktionsversuche. Nebst der generellen Durchführbarkeit wurde die Abhängigkeit der Temperatur, der Fluiddynamik und der Prozessfahrweise in- oder ausserhalb der Hohlfasermembranen (lumen-side / shell-side) ermittelt.

| Nr. | Protein | Volumen PEG-Phase [ml] | Volumen Puffer-Phase [ml] | K [10⁻⁶m/s] |
|---|---|---|---|---|
| 1 | Lysozym | 1500 | 300 | 7.56 |
| 2 | Lysozym | 1500 | 300 | 33.9 |
| 3 | Lysozym | 1500 | 400 | 30.8 |
| 4 | Lysozym | 1500 | 400 | 31.7 |
| 5 | Lysozym | 1875 | 500 | 34.7 |
| 6 | Lysozym | 1500 | 400 | 23.3 |
| 7 | BSA | 2000 | 400 | 10.8 |

Die erzielten Gesamtstoffdurchgangskoeffizienten K liegen zwischen 7,56x 10⁻⁶ m/s und 34,7x 10⁻⁶ m/s. Die Messungen von Dahuron und Cussler (Protein Extraction with Hollow Fibers, AIChE Journal (34/1), 1998) liegen bei 2,0x 10⁻⁶ m/s für Urease und 2,8x 10⁻⁷ m/s für Catalase, also ungefähr einen Faktor 10 tiefer. Dies ist ein Indiz dafür, dass sich mit dem erfindungsgemässen Verfahren zumindest ein Extraktionserfolg im Rahmen früherer Verfahren erreichen lässt, wobei aber der Verfahrensablauf wesentlich erleichtert und die Prozessstabilität wesentlich verbessert ist.

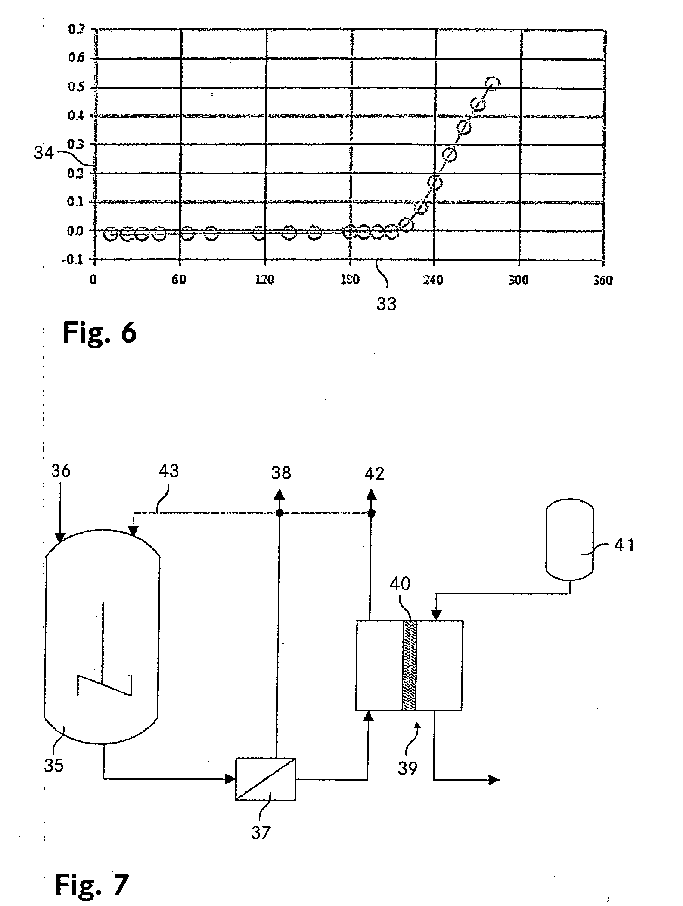

Im Rahmen des Versuchs 3 (vgl. Fig. 6) wurde das Tensid Tween® 20 erst nach drei Stunden Betrieb der Extraktionsanlage zur PEG-Phase gegeben. Innerhalb dieses Versuchszeitraumes konnte kein Protein in der Aufnehmerphase festgestellt werden. Erst nach der Zugabe von Tween® 20 konnte das Protein in der Aufnehmerphase nachgewiesen werden. Die Figur 6 zeigt den Konzentrationsverlauf von Lysozym in der Aufnehmerphase vor und nach der Zugabe von Tween® 20. Auf der horizontalen Achse 33 ist die Zeit in Minuten aufgetragen, auf der vertikalen Achse 34 die Konzentration der zu extrahierenden Komponente in der Aufnehmerphase in mg/ml. Vor der Zugabe des Tensids konnte das Protein nicht in der Aufnehmerphase nachgewiesen werden, obwohl unter den gegebenen Randbedingungen (d. h. ohne Tween® 20) im Schüttelversuch eine nachweisbare Verteilung dieser Wertkomponente zwischen den beiden Flüssigphasen erfolgte. Daraus folgt, dass bei der membrangestützten Extraktion unter diesen Bedingungen die beiden Flüssigphasen nicht miteinander kontaktiert werden können.

Die Verzögerung des Extraktionsbeginns nach dem Zugeben des Tensids (bei einer Minute) um knapp vierzig Minuten kann dadurch erklärt werden, dass sich das zugegebene Tween® 20 erst in der Versuchsanlage verteilen muss, um dann die Membranporen vollständig zu benetzen. Das sukzessive Ansteigen des Stofftransports zeigt, dass auch der Benetzungsprozess eine gewisse Zeit benötigt. Beim Auftropfen der benetzenden mit Tween@ 20 versetzten Phase auf eine hydrophobe Flachmembran konnte beobachtet werden, dass die poröse Membran erst nach etwa fünf Minuten klar und somit benetzt wurde. Der Extraktionsverlauf zeigt auch, dass sich, nachdem die Membran benetzt ist, ein stabiler Stofftransport einstellt. In diesem Fall beträgt der aus dem Extraktionsverlauf errechnete Gesamtstoffdurchgangskoeffizient 30.8x 10⁻⁶ m/s.

Dies zeigt deutlich, dass ein Extraktionsbetrieb erst durch die Zugabe von Tween® 20 ermöglicht wird. Im Gegensatz zu den oben erwähnten früheren Arbeiten (Dahuron und Cussler), bei welchen ein äusserer Druck der Lösungen auf beiden Seiten der Membran angelegt wurde, um die beiden wässrigen Lösungen mit annähernd gleichen Benetzungseigenschaften über eine hydrophobe Membran zu kontaktieren, war im Rahmen unserer Versuche lediglich ein Ventil auf der Seite der Puffer-Phase vorgesehen, um den transmembranen Druck auf ungefähr 40 mbar einzustellen, damit ein "Ausbluten" einer wässrigen Flüssigphase sicher vermieden werden kann. Für die Prozessstabilität selbst war dieser transmembrane Druck dagegen nicht notwendig.

Für den Fall von Toluol/Wasser-Systemen wurde bereits früher beschrieben, dass eine stabile Prozessfahrweise durch die wesentlichen Benetzungsunterschiede dieses Systems in einem weiten Druckbereich zwischen nahe 0 mbar und 3000 mbar möglich ist (W. Riedl, Membrangestützte Flüssig-Flüssig-Extraktion bei der Caprolactamherstellung, Shaker Verlag, Aachen, 2003). Dies rührt daher, dass nur ein sehr geringer Druck notwendig ist, um das Austreten der benetzenden Phase aus der Membranpore zu verhindern, jedoch ein sehr hoher Druck vorherrschen muss, um die nicht benetzende Flüssigkeit überhaupt in die Membranpore zu bringen. Aufgrund des Benetzungsunterschieds der beiden wässrigen Phasen sind diese Ergebnisse auch auf das erfindungsgemässe wässrige Zwei-Phasen-System übertragbar. Phasendurchbrüche sind also nur bei einem sehr hohen transmembranen Druck zu erwarten, so dass sich in der Regel eine aufwändige Bestimmung des transmembranen Drucks erübrigen dürfte. Im Vergleich zu bisher durchgeführten Arbeiten, bei denen der transmembrane Druck zur Ermöglichung der Extraktion genauestens eingestellt werden musste, ist hier lediglich das Anlegen eines geringen transmembranen Druckes auf der Puffer-Phase sinnvoll, um ein Ausbluten der PEG-Phase in die Puffer-Phase wirksam zu verhindern.

Beim Versuch 1 wurde für die PEG-Phase der Kreislauf ausserhalb der Hohlfasermembran (shell-side) gewählt, beim Versuch 2 zum Vergleich der Kreislauf innerhalb der Hohlfasermembran (lumen-side). Im Rahmen der Durchführung des Versuchs 5 wurde die Durchflussgeschwindigkeit seitens der PEG-Phase von zuvor 3.2x10⁻³ m/s auf 5.0x 10⁻³ m/s erhöht. Der Versuch 6 erfolgte nicht wie die anderen Versuche bei Raumtemperatur, sondern bei einer Temperatur von 36 °C.

Um die Eignung anderer wässriger Zwei-Phasen-Systeme und anderer Tenside zu untersuchen, wurden weitere Versuchsreihen durchgeführt.

Mit einem anderen wässrigen Zwei-Phasen-System, dem System PEG/Dextran/Wasser, wurde das Protein Lysozym membrangestützt extrahiert. Der Versuch wurde analog durchgeführt wie oben für das System PEG/Phosphatpuffer für die Versuche 2 bis 4 beschrieben. Auch hier konnte die Extraktion erst durch Zugabe des Tensids Tween® 20 erfolgreich und prozessstabil durchgeführt werden, wie der folgende Verlauf der Konzentration von Lysozym in der Pufferphase (Aufnehmerphase) zeigt:

| Zeit (min) | c (mg/ml) |
|---|---|
| 0 | 0.0000 |
| 10 | 0.0020 |
| 30 | 0.0416 |
| 60 | 0.2594 |
| 75 | 0.3890 |
| 93 | 0.5046 |
| 105 | 0.5568 |
| 125 | 0.6562 |

Der aus diesen Werten abgeleitete Gesamtstoffdurchgangskoeffizient K liegt mit 19,9x10⁻⁶ m/s in derselben Grössenordnung wie der in der ersten Versuchsreihe bestimmte Koeffizient mit dem System PEG/Phosphatpuffer. Die Durchführbarkeit des erfindungsgemässen Verfahrens ist also nicht an ein bestimmtes wässriges Zwei-Phasen-System gebunden.

Als Nächstes wurde der Einfluss verschiedener Tenside auf die Benetzungseigenschaften der Phasen verschiedener wässriger Zwei-Phasen-Systeme untersucht. Untersucht wurden - neben dem bereits beschriebenen Tween® 20 - nun auch die weiteren Tenside Triton® X-100 (CAS-Nr. 9002-93-1) und Brij® 35 P (CAS-Nr. 9002-92-0). Neben den bereits erwähnten ATPS wurde zusätzlich auch das System PEG / Natriumsulfat untersucht. Die wässrigen Zwei-Phasen-Systeme wurden in einem Scheidetrichter hergestellt.

Von den so hergestellten Systemen (mit und ohne Zugabe der oben genannten Tenside) wurden dann Auftropfversuche auf einer mikroporösen, hydrophoben Polymermembran (Typ Celgard ® 2400, vgl. die vorangegangenen Benetzungsversuche) durchgeführt. Das Ziel war hier festzustellen, ob die Lösungen, denen die oben genannten Tenside zugegeben wurden, auf der Membran verlaufen bzw. in die Membranmatrix (Membranporen) eindringen, und somit die Membran benetzen können, oder ob sie einen Tropfen auf der Membran ausbilden und somit, wie das ATPS-System PEG/Phosphatpuffer ohne Tensidzugabe, die Membran nicht benetzen.

Gegenüber den oben erwähnten Versuchen mit exakter Randwinkelvermessung wurde hier das Verhalten der Flüssigkeiten beim Auftropfen auf die Membran rein visuell begutachtet. Dies erlaubte bereits eine eindeutige Unterscheidung zwischen den Fällen, in welchen eine Lösung die Oberfläche der Membran benetzte und den Fällen, in welchen die Membran nicht benetzt wurde.

| ATPS | Tensid | c_{T} (Gew.%) | Flüssigphase benetzt hydrophobe Membran |
|---|---|---|---|
| PEG/Phosphatpuffer | - | - | nein; Tropfenbildung |
| PEG/Phosphatpuffer | Tween® 20 | 1,3 | ja |
| PEG/Phosphatpuffer | Triton® X-100 | 1,3 | ja |
| PEG/Phosphatpuffer | Brij® 35 P | 0,1 | ja |
| PEG/Na₂SO₄ | - | - | nein; Tropfenbildung |
| PEG/Na₂SO₄ | Tween® 20 | 1,3 | ja |
| PEG/Na₂SO₄ | Triton® X-100 | 1,3 | ja |
| PEG/Na₂SO₄ | Brij® 35 P | 0,1 | ja |
| PEG/Dextran | - | - | nein; Tropfenbildung |
| PEG/Dextran | Tween® 20 | 0,9 | ja |
| PEG/Dextran | Triton® X-100 | 0,9 | ja |
| PEG/Dextran | Brij® 35 P | 0,06 | ja |

Wie die Tabelle zeigt, führt die Zugabe bereits geringer Tensidkonzentration bei allen ATPS dazu, dass die Flüssigphase, der das Tensid zugesetzt wurde, die Testmembran benetzt. Ohne Zugabe eines Tensides benetzen die ATPS die Membran hingegen nicht.

Demnach ist die für die Durchführbarkeit des erfindungsgemässen Verfahrens wichtige unterschiedliche Benetzbarkeit der beiden Flüssigphasen auch mit den Tensiden Triton® X-100 und Brij® 35 P gegeben und damit nicht an ein bestimmtes Tensid gebunden.

### Verfahrenstechnische Realisierung

Im Folgenden wird eine beispielhafte verfahrenstechnische Realisierung des erfindungsgemässen Verfahrens skizziert. Diese ist in der Figur 7 schematisch dargestellt. Hier wird die membrangestützte Extraktion dazu eingesetzt, die in einem Fermentationsprozess hergestellten biologischen Wertprodukte zu extrahieren (zu "ernten").

In einen Fermenter 35, in dem eine auf wässrigen Lösungen basierende Umsetzung abgeschlossen ist, werden die benötigten Mengen an konzentrierter Puffer-Lösung, konzentrierter PEG-Lösung, Tween® 20 und Wasser zugegeben (Pfeil 36), um eine PEG-Phase des korrespondierenden wässrigen Zwei-Phasen-Systems zu erhalten. Durch einen vorgeschalteten Verfahrensschritt der Reinigung, wie z.B. eine Ultra- oder Mikrofiltration, werden nun die Zellreste in einer entsprechenden Vorrichtung 37 von der entstandenen Abgeberphase entfernt und wegtransportiert (Pfeil 38). Die geklärte Abgeberphase wird im Gegenstrombetrieb durch einen geeigneten Membrankontaktor 39, welcher auf der andern Seite der Membran 40 bereits mit der hergestellten Puffer-Aufnehmerphase durchspült wird, gepumpt. Bei optimaler Auslegung des Membrankontaktors 39 findet damit eine hohe Anreicherung des zu extrahierenden biologischen Materials in der Puffer-Phase statt, welche aus einem Vorlagegefäss 41 durch den Membrankontaktor 39 gepumpt wird. Die abgereicherte PEG-Phase wird nach dem Durchgang durch den Membrankontaktor wegtransportiert (Pfeil 42).

Müssen in dem Prozess die Zellen nicht zerstört werden, werden bei der Abtrennung der Zellen kreislauffähige Technologien eingesetzt. Wird die biologische Umsetzung zudem durch die ATPS Komponenten nicht gestoppt, ist ein kontinuierlicher Kreislaufprozess realisierbar, bei dem die aufkonzentrierte Zellbrühe zusammen mit der abgereicherten PEG-Phase aus dem Membrankontaktor 39 und frischen Edukten wieder in den Fermenter 35 gegeben werden (Pfeil 43).

Die Erfindung ist nicht auf die dargestellten Ausführungsbeispiele beschränkt. Die Resultate der umfassenden Versuchsreihe und der Vergleichsversuche zeigen, dass die Erfindung im Rahmen einer Vielzahl von ATPS durchführbar ist und dass eine Vielzahl von oberflächenaktiven Stoffen zum Modifizieren mindestens einer der Phasen eingesetzt werden kann. Das Verfahren kann zur Extraktion verschiedensten biologischen Materials verwendet werden.

Bei der Durchführung des Verfahrens und der Verfahrenstechnik sind ebenfalls grosse Variationsmöglichkeiten gegeben, insbesondere bezüglich der Wahl des verwendeten Membrankontaktors und der Versuchsanordnung. Beispielsweise können auf einfache Weise zusätzliche Verfahrensschritte zur Behandlung der beiden Phasen vor oder nach der eigentlichen Extraktion realisiert werden. Der Prozess kann auch in der Gleichstrom-Fahrweise realisiert werden, oder es können Verstärkungsverfahren eingesetzt werden.

Zusammenfassend ist festzustellen, dass die Erfindung ein dem eingangs genannten technischen Gebiet zugehörendes Verfahren zur Extraktion von Proteinen oder Peptiden schafft, welches einfach handhabbar und in grosstechnischem Massstab einsetzbar ist.

## Patentansprüche

1. Verfahren zur Extraktion von Proteinen oder Peptiden aus einer ersten wässrigen Lösung (1) in eine zweite wässrige Lösung (2), wobei zwischen den wässrigen Lösungen (1, 2) eine poröse Membran (3) mit einem maximalen Porendurchmesser von 2 µm angeordnet ist, **dadurch gekennzeichnet, dass** mindestens eine der wässrigen Lösungen (1, 2) durch Zugabe eines biokompatiblen und oberflächenaktiven Stoffs so modifiziert wird, dass sie im Bezug auf die verwendete Membran (3) eine gegenüber der anderen wässrigen Lösung unterschiedliche Benetzbarkeit aufweist.

2. Verfahren nach Anspruch 1, **dadurch gekennzeichnet, dass** die mindestens eine der wässrigen Lösungen (1, 2) so modifiziert wird, dass sie die verwendete Membran (3) benetzt und in deren Poren eindringen kann, während die andere wässrige Lösung die Membran nicht benetzt und somit nicht in deren Poren eindringt.

3. Verfahren nach Anspruch 1 oder 2, **dadurch gekennzeichnet, dass** die eine der wässrigen Lösungen (1) derart modifiziert wird, dass eine Differenz zwischen einem ersten Kontaktwinkel (δ) zwischen der ersten wässrigen Lösung (1) und der Oberfläche der Membran (3) und einem zweiten Kontaktwinkel (δ) zwischen der zweiten wässrigen Lösung (2) und der Oberfläche der Membran (3) mindestens 5°, bevorzugt mindestens 10°, beträgt.

4. Verfahren nach einem der Ansprüche 1 bis 3, **dadurch gekennzeichnet, dass** die poröse Membran hydrophob ist.

5. Verfahren nach Anspruch 4, **dadurch gekennzeichnet, dass** die eine der wässrigen Lösungen (1,2) derart modifiziert wird, dass ein Kontaktwinkel (δ) zwischen der modifizierten wässrigen Lösung (1) und einer Oberfläche der Membran (3) kleiner ist als 90°, bevorzugt kleiner als 50°.

6. Verfahren nach einem der Ansprüche 1 bis 5, **dadurch gekennzeichnet, dass** der Stoff ein Tensid ist.

7. Verfahren nach Anspruch 6, **dadurch gekennzeichnet, dass** der Stoff aus einem der folgenden Stoffe ausgewählt wird: Tween-20, Triton X-1 00, Brij 35 P.

8. Verfahren nach Anspruch 6 oder 7, **dadurch gekennzeichnet, dass** die eine der wässrigen Lösungen (1) derart modifiziert wird, dass eine Konzentration des Tensids in der modifizierten wässrigen Lösung (1) höchstens 2.5 Gew.%, bevorzugt weniger als 1.0 Gew.%, beträgt.

9. Verfahren nach einem der Ansprüche 1 bis 3, **dadurch gekennzeichnet, dass** die poröse Membran hydrophil ist und dass die eine der wässrigen Lösungen (1,2) derart modifiziert wird, dass ein Kontaktwinkel (δ) zwischen der modifizierten wässrigen Lösung (1) und einer Oberfläche der Membran (3) mindestens 90° ist.

10. Verfahren nach einem der Ansprüche 1 bis 9, **dadurch gekennzeichnet, dass** als Stoff zum Modifizieren mindestens der einen wässrigen Lösung (1) ein Stoff ausgewählt wird, welcher sich verglichen mit der anderen wässrigen Lösung (2) stark bevorzugt in der zu modifizierenden wässrigen Lösung (1) anreichert.

11. Verfahren nach einem der Ansprüche 1 bis 10, **dadurch gekennzeichnet, dass** die Membran (3) eine poröse Flach-, Kapillar- oder Hohlfasermembran ist.

12. Verfahren nach einem der Ansprüche 1 bis 11, **dadurch gekennzeichnet, dass** eine der wässrigen Lösungen (1, 2) ein Polymer enthält, insbesondere PEG (Polyethylenglykol) oder Dextran, und dass in der anderen der Lösungen (2, 1) vorrangig ein Salz oder ein Phosphatpuffer gelöst ist.

13. Verfahren nach einem der Ansprüche 1 bis 11, **dadurch gekennzeichnet, dass** die erste wässrige Lösung (1; 2) ein erstes Polymer enthält und dass die zweite wässrige Lösung (2; 1) ein zweites Polymer enthält, wobei die Polymere wasserlöslich und nicht oder nur geringfügig miteinander mischbar sind und wobei die zwei Polymere bevorzugt Polyethylenglykol (PEG) und Dextran sind.

14. Verfahren nach einem der Ansprüche 1 bis 13, **dadurch gekennzeichnet, dass** mindestens eine der wässrigen Lösungen (1, 2) vor oder nach der Extraktion destillativ, mittels Membranverfahren, Flockung und/oder durch andere geeignete Trennverfahren in ihren physikalischen, chemischen und/oder thermodynamischen Eigenschaften verändert wird.

15. Verwendung eines biokompatiblen Stoffs in einem Verfahren gemäss einem der Ansprüche 1 bis 14 zur Extraktion von Proteinen oder Peptiden aus einer ersten wässrigen Lösung (1) in eine zweite wässrige Lösung (2), wobei zwischen den wässrigen Lösungen (1, 2) eine poröse Membran (3) angeordnet ist, **dadurch gekennzeichnet, dass** mindestens eine der wässrigen Lösungen (1, 2) durch Zugabe des Stoffs so modifiziert wird, dass sie im Bezug auf die verwendete Membran (3) eine gegenüber der anderen wässrigen Lösung unterschiedliche Benetzbarkeit aufweist.

## Claims

1. A process for the extraction of proteins or peptides from a first aqueous solution (1) into a second aqueous solution (2), wherein a porous membrane (3) having a maximum pore diameter of 2 µm is arranged between the aqueous solutions (1,2), **characterized in that** at least one of the aqueous solutions (1, 2) is so modified by the addition of a biocompatible surface-active substance that in relation to the membrane (3) used it has a different wettability compared to the other aqueous solution.

2. A process according to claim 1, **characterized in that** the at least one of the aqueous solutions (1, 2) is so modified that it wets the membrane (3) used and can penetrate into the pores thereof, while the other aqueous solution does not wet the membrane and thus does not penetrate into the pores thereof.

3. A process according to claim 1 or claim 2, **characterized in that** the one of the aqueous solutions (1) is so modified that a difference between a first contact angle (δ) between the first aqueous solution (1) and the surface of the membrane (3) and a second contact angle (δ) between the second aqueous solution (2) and the surface of the membrane (3) is at least 5°, preferably at least 10°.

4. A process according to any one of claims 1 to 3, **characterized in that** the porous membrane is hydrophobic.

5. A process according to claim 4, **characterized in that** the one of the aqueous solutions (1, 2) is so modified that a contact angle (δ) between the modified aqueous solution (1) and a surface of the membrane (3) is less than 90°, preferably less than 50°.

6. A process according to any one of claims 1 to 5, **characterized in that** the substance is a tenside.

7. A process according to claim 6, **characterized in that** the substance is selected from one of the following substances: Tween-20, Triton X-100, Brij 35 P.

8. A process according to claim 6 or claim 7, **characterized in that** the one of the aqueous solutions (1) is modified in such a way that a concentration of the tenside in the modified aqueous solution (1) is at the most 2.5 % by weight, preferably less than 1.0 % by weight.

9. A process according to any one of claims 1 to 3, **characterized in that** the porous membrane is hydrophilic and **in that** the one of the aqueous solutions (1, 2) is modified in such a way that a contact angle (δ) between the modified aqueous solution (1) and a surface of the membrane (3) is at least 90°.

10. A process according to any one of claims 1 to 9, **characterized in that** as the substance for modifying at least the one aqueous solution (1) a substance is selected which, compared to the other aqueous solution (2), builds up highly preferably in the aqueous solution (1) to be modified.

11. A process according to any one of claims 1 to 10, **characterized in that** the membrane (3) is a porous flat, capillary or hollow fibre membrane.

12. A process according to any one of claims 1 to 11, **characterized in that** one of the aqueous solutions (1, 2) contains a polymer, especially PEG (polyethyleneglycol) or dextran, and **in that** in the other of the solutions (2, 1) a salt or a phosphate buffer is predominantly dissolved.

13. A process according to any one of claims 1 to 11, **characterized in that** the first aqueous solution (1; 2) contains a first polymer and **in that** the second aqueous solution (2; 1) contains a second polymer, the polymers being water-soluble and only slightly or not at all mixable with each other, and the two polymers preferably being polyethyleneglycol (PEG) and dextran.

14. A process according to any one of claims 1 to 13, **characterized in that** at least one of the aqueous solutions (1, 2), before or after extraction, is altered in its physical, chemical and/or thermodynamic properties by distillation, by means of a membrane process, flocculation and/or by means of other suitable separating processes.

15. The use of a biocompatible substance in a process according to any one of claims 1 to 14 for the extraction of proteins or peptides from a first aqueous solution (1) into a second aqueous solution (2), a porous membrane (3) being arranged between the aqueous solutions (1, 2), **characterized in that** at least one of the aqueous solutions (1, 2) is so modified by the addition of the substance that in relation to the membrane used (3) it has a different wettability compared to the other aqueous solution.

## Revendications

1. Procédé d'extraction de protéines ou de peptides d'une première solution aqueuse (1) dans une seconde solution aqueuse (2), une membrane poreuse (3) ayant un diamètre de pores maximal de 2 µm étant placée entre les solutions aqueuses (1, 2), **caractérisé en ce qu'**au moins l'une des solutions aqueuses (1, 2) est modifiée en ajoutant une matière biocompatible et tensioactive de manière à présenter par rapport à la membrane (3) employée une mouillabilité différente comparé à l'autre solution aqueuse.

2. Procédé selon la revendication 1, **caractérisé en ce que** la au moins une des solutions aqueuses (1, 2) est modifiée de manière à mouiller la membrane (3) employée et à pouvoir pénétrer dans ses pores, tandis que l'autre solution aqueuse ne mouille pas la membrane et donc ne pénètre pas dans ses pores.

3. Procédé selon la revendication 1 ou 2, **caractérisé en ce que** l'une des solutions aqueuses (1) est modifiée de manière à obtenir une différence d'au moins 5 degrés, de préférence d'au moins 10 degrés entre un premier angle de contact (δ) entre la première solution aqueuse (1) et la surface de la membrane (3) et entre un second angle de contact (δ) entre la seconde solution aqueuse (2) et la surface de la membrane (3).

4. Procédé selon l'une des revendications 1 à 3, **caractérisé en ce que** la membrane poreuse est hydrophobe.

5. Procédé selon la revendication 4, **caractérisé en ce que** l'une des solutions aqueuses (1, 2) est modifiée de manière à ce qu'un angle de contact (δ) entre la solution aqueuse (1) modifiée et une surface de la membrane (3) soit inférieur à 90 degrés, de préférence, inférieur à 50 degrés.

6. Procédé selon l'une des revendications 1 à 5, **caractérisé en ce que** la matière est un tensioactif.

7. Procédé selon la revendication 6, **caractérisé en ce que** la matière est choisie parmi les matières suivantes : Tween-20, Triton X-100 et Brij 35 P.

8. Procédé selon la revendication 6 ou 7, **caractérisé en ce que** l'une des solutions aqueuses (1) est modifiée de manière à obtenir une concentration du tensioactif dans la solution aqueuse (1) modifiée d'au plus 2,5 % en poids, de préférence, inférieure à 1,0 % en poids.

9. Procédé selon l'une des revendications 1 à 3, **caractérisé en ce que** la membrane poreuse est hydrophile et **en ce que** l'une des solutions aqueuses (1, 2) est modifiée de manière à avoir un angle de contact (δ) d'au moins 90 degrés entre la solution aqueuse (1) modifiée et une surface de la membrane (3).

10. Procédé selon l'une des revendications 1 à 9, **caractérisé en ce que** l'on choisit comme matière pour modifier au moins la première solution aqueuse (1) une matière qui, comparé à l'autre solution aqueuse (2), se concentre de manière fortement préférée dans la solution aqueuse (1) à modifier.

11. Procédé selon l'une des revendications 1 à 10, **caractérisé en ce que** la membrane (3) est une membrane poreuse à fibres plates, à fibres capillaires ou à fibres creuses.

12. Procédé selon l'une des revendications 1 à 11, **caractérisé en ce qu'**une des solutions aqueuses (1, 2) contient un polymère, notamment du PEG (polyéthylène glycol) ou du dextrane et **en ce que** l'on dissout préalablement dans l'autre des solutions (2, 1) un sel ou un tampon phosphate.

13. Procédé selon l'une des revendications 1 à 11, **caractérisé en ce que** la première solution aqueuse (1; 2) contient un premier polymère et **en ce que** la seconde solution aqueuse (2; 1) contient un second polymère, les polymères étant hydrosolubles et pas du tout ou à peine miscibles l'un dans l'autre et les deux polymères étant de préférence le polyéthylène glycol (PEG) et le dextrane.

14. Procédé selon l'une des revendications 1 à 13, **caractérisé en ce que** l'on modifie par distillation les propriétés physiques, chimiques et/ou thermodynamiques d'au moins une des solutions aqueuses (1, 2) avant ou après l'extraction au moyen d'un procédé à membrane, d'une floculation et/ou au moyen d'autres procédés de séparation adaptés.

15. Utilisation d'une matière biocompatible dans un procédé selon l'une des revendications 1 à 14 pour l'extraction de protéines ou de peptides d'une première solution aqueuse (1) dans une seconde solution aqueuse (2), une membrane poreuse (3) étant placée entre les solutions aqueuses (1, 2), **caractérisée en ce qu'**au moins l'une des solutions aqueuses (1, 2) est modifiée en ajoutant la matière de manière à présenter par rapport à la membrane (3) employée une mouillabilité différente comparé à l'autre solution aqueuse.
